# EUROPEAN PATENT APPLICATION

(11) **EP 4 428 602 A1**
(43) Date of publication of application: **11.09.2024**
(21) Application number: 22889921.7
(22) Date of filing: 31.10.2022
(51) Int. Cl.: G02C 7/04, G02C 7/10

(54) **HYDROGEL**

(30) Priority: 02.11.2021 JP 2021179262
(71) Applicant: Seed Co., Ltd., Tokyo 113-8402 (JP)
(72) Inventor: YAMAZAKI, Yoshiko, Tokyo 113-8402 (JP); ARAI, Ritsuko, Tokyo 113-8402 (JP); KAWAUCHIYA, Chiaki, Tokyo 113-8402 (JP); SATO, Takao, Tokyo 113-8402 (JP)
(74) Representative: Schulz Junghans Patentanwälte PartGmbB
(86) International application number: PCT/JP2022/040592
(87) International publication number: WO 2023/080101

(57) **Abstract**

The purpose of the present invention can be achieved by a hydrogel containing amorphous carbon. The hydrogel can be used as an ophthalmic hydrogel, and when the ophthalmic hydrogel is in a contact lens form, it is preferable that the amorphous carbon is arranged in a circular pattern on the surface and/or inside except for the optical area of the hydrogel, or when the ophthalmic hydrogel is in an annular or oval form, it is preferable that the amorphous carbon is uniformly arranged throughout the surface and/or inside of the hydrogel.

## Description

### Technical Field

The present invention relates to hydrogels, and more particularly to hydrogels containing amorphous carbon.

### Background Art

Generally, medication treatment, one of the methods of treatment for various diseases, may be suspended when side effects are significant, or the medication may be changed if alternative treatment is available, etc. However, when symptoms of side effects are not severe or alternative treatment is not available, the medication is often continued. From the viewpoint of improving the quality of life (QOL) of patients, it is an important issue to suppress the occurrence of side effects or to alleviate symptoms when side effects have occurred.

One of the medications prescribed in many cancer treatments is TS-1, a known anticancer agent (see Non-Patent Document 1). TS-1 is a prodrug that is comprised of tegafur, gimerasil, and potassium oterasil, and fluorouracil (5FU), which is produced when tegafur is metabolized in the body, expresses anticancer activity. One of the side effects of TS-1 medication treatment is lacrimation caused by lacrimal duct obstruction. The mechanism by which the side effect occur is thought to be that 5FU produced by the metabolism of tegafur migrates from plasma into the tear fluid and contacts the tear duct wall, causing thickening of the squamous epithelium and fibrosis of the stroma, resulting in narrowing or obstruction of the tear duct and blockage of tear discharge, which causes the tear fluid to overflow from the eye. When symptoms of lacrimation occur, there is no established method of prevention, although there is concern that if not treated early, the sequelae of lacrimation may remain and significantly impair QOL. Paclitaxel and docetaxel, as well as TS-1, are also used as anticancer agents. In addition to lacrimation and other lacrimal duct disorders, cases of corneal and optic nerve disorders caused by exposure to tear fluid containing the drug and its metabolites on the eye surface have also been reported.

In order to prevent the occurrence of lacrimation, which is a side effect, during medication treatment, it is important to remove causative substances that cause lacrimal duct obstruction so that they do not remain in the tear fluid. However, at present, the main method of symptomatic therapy is to physically wash out and remove the tears by applying saline or other ophthalmic drops when the symptoms of lacrimation occur, and thus preventive treatment is required.

For example, Patent Document 1 discloses a method of removing 5FU by chemically binding it to a heterocyclic compound which is a component of the ophthalmic lens base material, before the 5FU in the tear fluid develops side effects.

It is known that medicinal carbon is used for gastrointestinal decontamination, which is one of the countermeasures to be taken in case of drug overdose or accidental ingestion of toxic substances when the object is not absorbed into the digestive tract (see Non-Patent Document 2). This medicinal carbon is an antidiarrheal or an intestinal regulator for diarrhea, adsorption of gases produced by abnormal fermentation in the digestive tract, adsorption and detoxification in autointoxication and drug poisoning. Medicinal carbon is administered orally with the activated charcoal itself.

### Citation List

### Patent Documents

Patent Document 1: JP 2018-203832 A

### Non-Patent Documents

Non-Patent Document 1: S-1 TAIHO OD Tablet Package Insert
Non-Patent Document 2: Medicinal carbon Package Insert

### Summary of the Invention

### Problems to be Solved by the Invention

However, the method disclosed in Patent Document 1 is a mechanism to capture 5FU into the ophthalmic lens base material by chemical bonding using the heterocyclic compound, which has a structure that can form base pairs with 5FU in the tear fluid, as a component of the hydrogel which is the ophthalmic lens base material. Thus, the combination of the compound to be captured and the ophthalmic lens base material is limited. Therefore, when different compounds are captured by the same mechanism, the constituents of the ophthalmic lens base material must be changed according to the chemical structure of the target compound, which poses a problem to its versatility.

Under the circumstances described above, it is an object of the present invention to provide a hydrogel with excellent versatility that can capture a plurality of target compounds, regardless of components of base materials, when a predefined compound derived from a medication treatment drug that is remained in tear fluid is captured by an ophthalmic hydrogel.

### Means of Solving the Problems

While investigating a method for removing multiple target compounds including 5FU, from the tear fluid using a single ophthalmic hydrogel, the inventors focused on physical adsorption instead of chemical adsorption, which had been used in the prior art. In particular, while evaluating the physical adsorption of 5FU using various substances, we found that 5FU was more effectively adsorbed when amorphous carbon was used than when other substances were used. The present invention has been completed on the basis of the findings and successful examples that were first found or obtained by the present inventors.

According to the present invention, there are provided a hydrogel of the following embodiments.
[1] A hydrogel comprising amorphous carbon.
[2] The hydrogel according to [1], wherein the hydrogel is an ophthalmic hydrogel.
[3] The hydrogel according to [2], wherein the ophthalmic hydrogel is in a contact lens form, and the amorphous carbon is arranged in a circular pattern on the surface and/or inside except for the optical area of the hydrogel.
[4] The hydrogel according to [2], wherein the ophthalmic hydrogel is in an annular or approximately oval form, and the amorphous carbon is uniformly arranged throughout the surface and/or inside of the hydrogel.
[5] The hydrogel according to any one of [1] to [4], wherein the amorphous carbon is physically capable of adsorbing a predefined compound.

### Effects of the Invention

According to the hydrogel of the present invention, the amorphous carbon in the hydrogel can physically adsorb a plurality of target compounds including 5FU, etc. According to the hydrogel of the present invention, for example, when the hydrogel is for ophthalmic use, the amorphous carbon is physically capable of adsorbing a plurality of target compounds, allowing a single hydrogel base material to capture a plurality of target compounds regardless of the components of the hydrogel base material, thus providing excellent versatility. As the result, by wearing the ophthalmic hydrogel of the present invention, for example, 5FU that remains in the tear fluid is physically adsorbed to the amorphous carbon, so that 5FU is removed from the tear fluid. It is expected to prevent the occurrence of lacrimation, which is one of the side effects.

According to the hydrogel of the present invention, when the hydrogel is for ophthalmic use, especially when the ophthalmic hydrogel is in a normal contact lens form and the amorphous carbon is arranged in a circular pattern on the surface and/or inside except for the optical area of the hydrogel, or when the ophthalmic hydrogel is in an annular form with an opening in an optical center including the optical area, or is in an approximately oval form suitable for use by being inserted between a low eyelid and an eyeball. The amorphous carbon is uniformly arranged on the surface and/or inside of the ophthalmic hydrogel to maintain the quality of vision (QOV) when the ophthalmic hydrogel is worn (i.e., visibility is not deteriorated.).

### Brief description of drawing

[Figure 1] A perspective view (photographic image) from above the front surface side showing an example of an ophthalmic hydrogel (contact lens form) according to one embodiment of the present invention.
[Figure 2] A perspective view (photographic image) from above the base surface side of the ophthalmic hydrogel showing in Fig.1.
[Figure 3] A perspective view (photographic image) from above the front surface side showing another example of an ophthalmic hydrogel (annular form) according to one embodiment of the present invention.
[Figure 4] A perspective view (photographic image) from above the base surface side of the ophthalmic hydrogel showing in Fig.3.
[Figure 5] A perspective view (photographic image) from above the front surface side showing another example of an ophthalmic hydrogel (approximately oval form) according to one embodiment of the present invention.

### Description of Embodiments

While each embodiment of the present invention will now be described in detail, the present invention may take various forms to the extent that its objective can be achieved.

Unless otherwise specified, each term used herein is used in the meaning commonly used by those skilled in the art, in particular in the food field, and should not be construed to have any meaning that is unduly limiting. Also, any speculations and theories herein are made on the basis of the knowledge and experiences of the present inventors and as such, the present invention is not bound by any such speculations and theories.

The term "and/or" as used herein means either any one of, any combination of two or more of, or combination of all of listed related items.

The wording "to" for indicating a range of values is intended to include values preceding and following the wording; for example, "0% to 100%" means a range from 0% or more and 100% or less.

"Monomer" is a term paired with "polymer" and means a molecule that can be polymerized by polymerizable groups in the molecule and can be used as a component of a polymer.

"Polymer" means a molecule formed by polymerization of monomers.

The term "(meth)acrylate" is a generic term that encompasses both acrylate and methacrylate.

### [Hydrogel]

The hydrogel according to one embodiment of the present invention contains amorphous carbon and at least hydrophilic monomer as a base component. Hydrogels can swell by containing water inside (hydration swelling) due to the presence of hydrophilic monomers.

In the hydrogel according to one embodiment of the present invention, it is particularly useful as an ophthalmic material that is directly attached to the eye. The structure of such ophthalmic hydrogels is not particularly limited as long as QOV is ensured. For example, there are normal contact lens form, annular form with an aperture at the optical center including the optical area of the contact lens, and approximately oval form suitable for insertion between the lower eyelid and eyeball.

According to the hydrogel according to one embodiment of the present invention, the amorphous carbon in the hydrogel is physically capable of adsorbing a plurality of target compounds including 5FU, etc. Thus, the ophthalmic hydrogel can capture a plurality of predefined compounds derived from a medication treatment drug that remains in the tear fluid.

### [Amorphous carbon]

Amorphous carbon is an allotrope of carbon, other than diamond and graphite, that does not exhibit a distinct crystalline state. Examples of such amorphous carbon include soot, charcoal, coke, and carbon black. Amorphous carbon contained in the hydrogel according to one embodiment of the present invention is not particularly limited, but powdered activated carbon and carbon black with nano-sized pore structure are preferably used from the viewpoint of dispersibility in the raw material solution of hydrogels. In the present invention, considering dispersibility in various solutions, the average particle diameter of powdered activated carbon and carbon black is 500 nm or less, more preferably 300 nm or less.

The amorphous carbon is preferably contained at a ratio of 1.0% by mass to 20% by mass in the production method (1) described below, and at a ratio of 0.1% by mass to 1.0% by mass in the production method (2) described below, with respect to the total component of hydrogel.

The hydrogel according to one embodiment of the present invention includes, for example, hydrogel formed using hydrophilic monomers, hydrogel formed using hydrophilic monomers with hydrophobic or crosslinking monomers, or both.

The hydrophilic monomers are not limited as long as they have hydrophilic groups and (meth)acrylic or vinyl groups in the molecule, but examples include hydroxymethyl (meth)acrylate, 2-hydroxyethyl (meth)acrylate, 2-hydroxypropyl (meth)acrylate, 2,3-dihydroxypropyl (meth)acrylate, 2-polyethylene glycol (meth)acrylate, acrylamide, 2-polypropylene glycol (meth)acrylate, N,N-dimethylmethacrylamide, N- vinylpyrrolidone (NVP), N,N-dimethylacrylamide (DMAA), (meth)acrylic acid, polyethylene glycol mono(meth)acrylate, glyceryl (meth)acrylate (glycerol (meth)acrylate), N-vinyl-N-methylacetamide, N-vinyl-N-ethylacetamide, N-vinyl-N-ethylformamide, and N-vinylformamide. To obtain the hydrogel with the desired properties, the above hydrophilic monomers can be used alone or in a mixture of two or more.

The hydrophobic monomers include, for example, trifluoroethyl (meth)acrylate, methacrylamide, siloxanil (meth)acrylate, methyl (meth)acrylate, n-butyl (meth)acrylate, tert-butyl (meth)acrylate, benzyl (meth)acrylate, ethyl hexyl (meth)acrylate, lauryl (meth)acrylate, etc. To obtain the hydrogel with the desired properties, the above hydrophobic monomers can be used alone or in a mixture of two or more.

The crosslinkable monomers include, for example, (meth)acrylate crosslinkable monomers such as ethylene glycol di(meth)acrylate, diethylene glycol di(meth)acrylate, triethylene glycol di(meth)acrylate, propylene glycol di(meth)acrylate, dipropylene glycol di(meth)acrylate, trimethylolpropane tri(meth)acrylate, pentaerythritol tri(meth)acrylate, pentaerythritol tetra(meth)acrylate, dipentaerythritol hexa(meth)acrylate; vinyl crosslinkable monomers such as allyl methacrylate, diallyl maleate, diallyl fumarate, diallyl succinate, diallyl phthalate, triallyl cyanurate, triallyl isocyanurate, diethylene glycol bisallyl carbonate, triallyl phosphate, triallyl trimellitate, diaryl ether, N,N -diarylmelamine, divinylbenzene. To obtain the hydrogel with the desired properties, the above crosslinkable monomers can be used alone or in a mixture of two or more. For the crosslinkable monomers, bifunctional crosslinkable monomers are more preferably used.

The crosslinkable monomer is contained at a ratio of 5% by mass or less to the total components of the hydrogel, more preferably 3% by mass or less. If the content of the crosslinkable monomers exceeds 5% by mass, the crosslinking action becomes stronger, and especially in the ophthalmic hydrogel, the flexibility may decrease and the practicality of applications such as contact lens applications may be inferior.

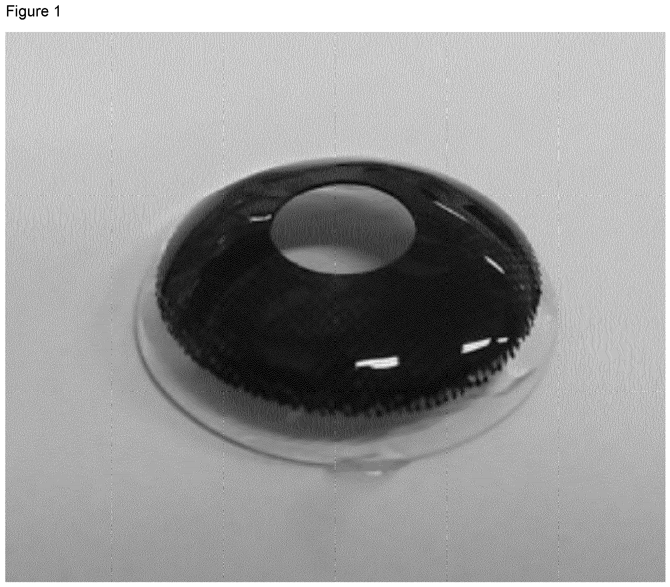

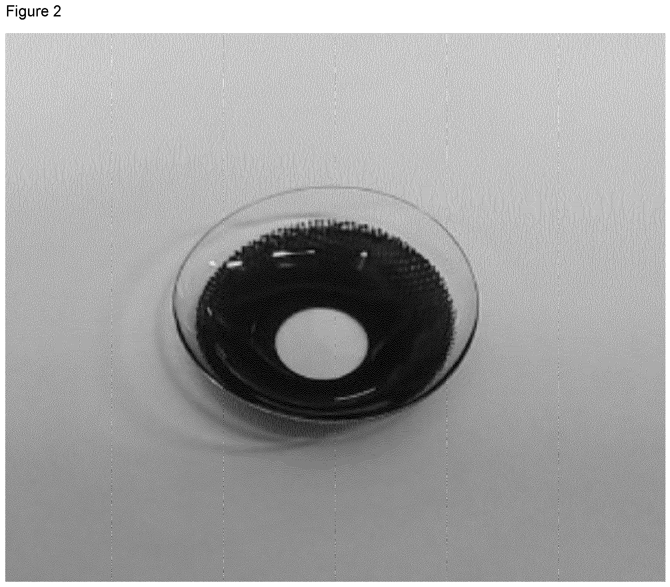

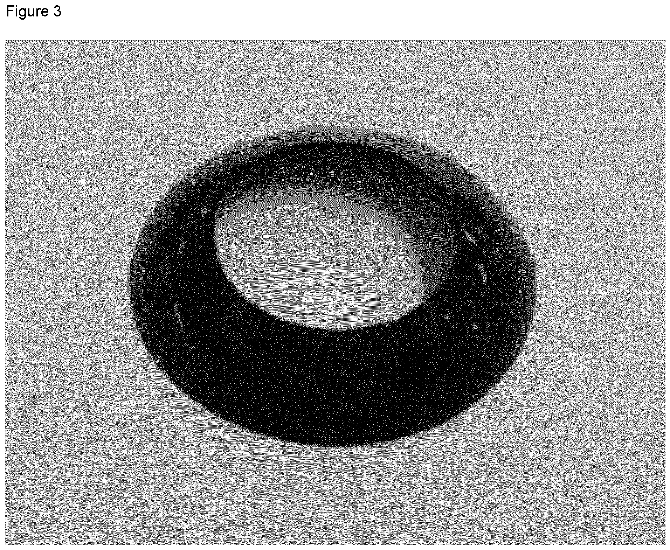

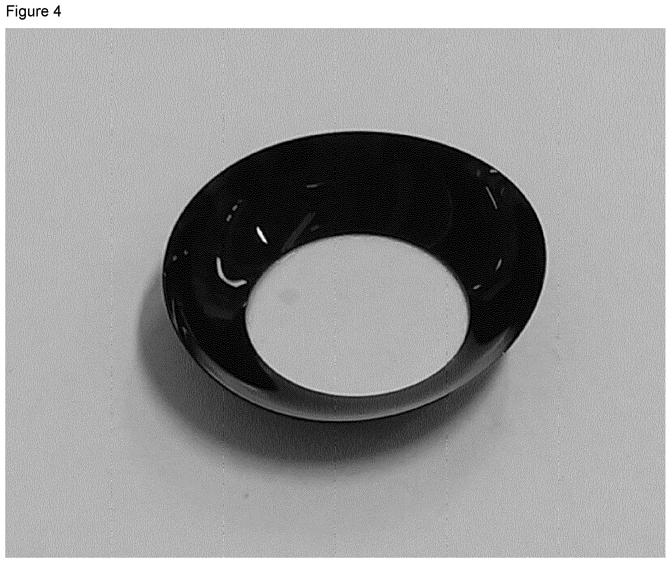

When the structure of the ophthalmic hydrogel according to one embodiment of the present invention is in the normal contact lens form for example, as shown in Figs. 1 and 2, it is preferable from viewpoint of QOV that the amorphous carbon is arranged in a circular pattern on the surface and/or inside except for the optical area. Further, when the structure of the ophthalmic hydrogel according to one embodiment of the present invention is in the annular form having the opening at the optical center including the optical area, as shown in Figs. 3 and 4, or when the structure of the ophthalmic hydrogel is in the approximately oval form that is suitable for insertion between the lower eyelid and the eyeball, it is preferable that the amorphous carbon is uniformly arranged throughout the surface and/or inside.

In the present invention, the optical area refers to the central portion of the lens, which corresponds to the diameter of the pupil (about 6 mm).

### [Production Method]

As for the production method of the hydrogel according to one embodiment of the present invention, any known method can be employed as long as the hydrogel containing amorphous carbon can be obtained.

For example, when the hydrogel according to one embodiment of the present invention is for ophthalmic use, the following methods (1) and (2) can be used to arrange the amorphous carbon on the surface and/or inside of the ophthalmic hydrogel.
(1) Method of uniformly dispersing amorphous carbon on a surface and/or inside of the ophthalmic hydrogel
(2) Method of forming a layer with amorphous carbon on a surface and/or inside of the ophthalmic hydrogel

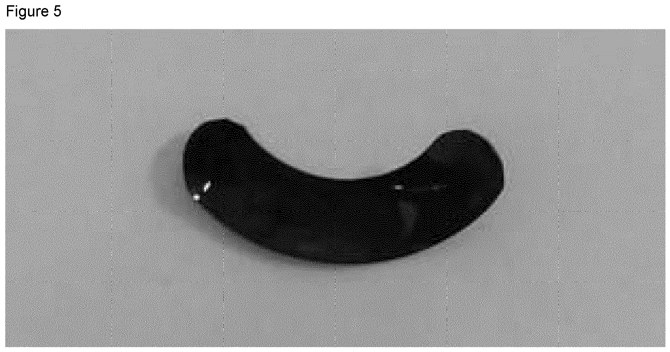

In the method (1) above, an amorphous carbon/material monomer solution obtained by dispersing amorphous carbon in a raw monomer solution contained as a component of the hydrogel base material serving as a carrier of amorphous carbon is subjected to copolymerization reaction in a shaping mold for forming ophthalmic hydrogel to obtain the ophthalmic hydrogel in which the amorphous carbon is uniformly arranged throughout a surface and/or inside. This method (1) is suitable when the structure of the ophthalmic hydrogel is in the annular form as shown in Figs. 3 and 4, or the approximately oval form as shown in Fig. 5.

In the method (1) above, since the dispersibility of the amorphous carbon improves when the raw material monomer solution has a predefined viscosity, a polymer compound having solubility in the raw material monomer can be added to the raw material monomer solution.

The polymer compound is not limited as long as it has solubility in the raw monomer. Examples include polyvinyl alcohol and polyvinyl pyrrolidone.

In the method (2) above, the position of the amorphous carbon arrangement can be controlled. In the present invention, when the structure of the ophthalmic hydrogel is formed into the contact lens form as shown in Figs. 1 and 2, the method (2) above is preferred because it is necessary to attach specific optical characteristics when wearing.

The method (2) above is explained in detail below.

### (2-1) Production method of ophthalmic hydrogel with amorphous carbon on a surface

After forming a layer consisting of amorphous carbon dispersion solution on a surface of male and/or female mold for forming ophthalmic hydrogel, the raw monomer solution contained as a constituent of the hydrogel base material is injected into the female mold so that the hydrogel base material is formed on the said layer. After the male mold is fitted, a copolymerization reaction is performed to obtain an ophthalmic hydrogel in which the amorphous carbon is arranged on the front side surface and/or base side surface.

### (2-2) Production method of ophthalmic hydrogel with amorphous carbon on an inside

After injecting a first raw monomer solution contained as a component of a first hydrogel base material into a female mold for forming ophthalmic hydrogel and fitting the male mold, the mold is subjected to a copolymerization reaction to form a layer consisting of the first hydrogel base material, and after the copolymerization reaction, the mold is separated after it has cooled to room temperature to obtain the mold in which the layer is formed on either the female or male mold. Thereafter, a layer consisting of amorphous carbon dispersion solution is further formed on said layer, and a second raw monomer solution, which is contained as a component of a second hydrogel base material, is injected into the female mold so that the second hydrogel base material is formed on top of said layer, and after fitting the male mold, it is subjected to copolymerization reaction to form the second hydrogel base material to obtain the ophthalmic hydrogel in which amorphous carbon is arranged inside.

By combining the methods (2-1) and (2-2) above, an ophthalmic hydrogel with amorphous carbon only on the surface, an ophthalmic hydrogel with amorphous carbon only inside, or an ophthalmic hydrogel with amorphous carbon inside and on the surface can be produced.

In the methods (2-1) and (2-2) above, the ophthalmic hydrogel obtained by forming the layer consisting of the amorphous carbon dispersion solution so as not to contact the optical area of the ophthalmic hydrogel can maintain constant optical properties.

In the methods (2-1) and (2-2) above, the formation of the layer consisting of amorphous carbon dispersion solution can employ methods normally used to form thin film layers on the surface of contact lenses and eyeglass lenses. Such layer forming methods include, for example, pad printing, spin casting, dipping, etc. In the present invention, the pad printing method is preferred from the viewpoint of controlling shapes of the layer consisting of the amorphous carbon dispersion solution. In forming the layer consisting of the amorphous carbon dispersion solution, it is preferable to dry the layer after forming it in the manner described above.

In the methods (2-1) and (2-2) above, the amorphous carbon dispersion solution has a predefined viscosity, which improves the dispersibility of the amorphous carbon in the solution and the film formation on the surface of the mold or the first hydrogel base material.

In order for the amorphous carbon dispersion solution to obtain the predefined viscosity, a polymer compound is contained in the amorphous carbon dispersion solution. One method to contain polymer compounds is to mix amorphous carbon with pre-prepared polymer compound solution. One method to mix amorphous carbon with polymer compound solutions include, but are not limited to, the use of a vortex mixer and a magnetic stirrer.

In the hydrogel according to one embodiment of the present invention, a hydrophilic monomer is used as a base material component, but when considering the affinity between the base material component and the amorphous carbon dispersion solution, it is preferable that the amorphous carbon dispersion solution also have hydrophilic properties. Therefore, the polymer compound solution used is also hydrophilic to obtain a suitable amorphous carbon dispersion solution. Such hydrophilic polymer compound solutions include poly (2-hydroxyethyl methacrylate) solution (hereinafter also referred to as "p-HEMA solution"), poly (2-hydroxyethyl acrylate) solution (hereinafter referred to as "p-HEA solution"), Polyvinyl alcohol solution (hereinafter referred to as "PVA solution"), Polyvinylpyrrolidone solution (hereinafter referred to as "PVP solution"), etc. One or more of these can be used alone or in a mixture of two or more.

In the production method of (1) or (2) above, when subjecting the raw material monomer solution to a copolymerization reaction, a thermal polymerization initiator typified by peroxides and azo compounds, a photopolymerization initiator, etc.can be added. Such polymerization initiators include peroxide polymerization initiators such as lauroyl peroxide, cumene hydroperoxide, and benzoyl peroxide, which are common radical polymerization initiators; azo polymerization initiators such as azobisdimethylvaleronitrile and azobisisobutyronitrile. One or more these can be used alone or in a mixture of two or more.

The amount of polymerization initiator added is not limited as long as it is sufficient to promote the copolymerization reaction of the monomers. For example, 10 ppm to 10000 ppm is preferred relative to the total amount of the raw monomer solution. When the amount of polymerization initiator added is less than 10 ppm, the polymerization reaction may be insufficient and the ophthalmic hydrogel with appropriate strength may not be obtained. On the other hand, when the amount of polymerization initiator added exceeds 10000 ppm, the polymerization rate is fast, resulting in a non-uniform reaction, and there is a risk that a polymer with appropriate strength cannot be obtained.

In the production method of (1) or (2) above, the copolymerization reaction is performed by placing the components of the hydrogel with the polymerization initiator in a metal, glass, plastic, or other mold, sealing the mold, and raising the temperature in the range of 25°C to 120°C in stages or continuously in a constant temperature chamber or the like to complete the polymerization in 5 hours to 120 hours, or by irradiating with ultraviolet rays, electron beams, gamma rays, etc. Solution polymerization can also be employed by adding water or an organic solvent to the constituent of the hydrogel.

In the production method of (1) or (2) above, after the copolymerization reaction, the mold is cooled to room temperature, the resulting copolymer is peeled from the mold, cut and polished as necessary, and then hydrated and swollen to obtain hydrogel. Examples of liquids (swollen solutions) used include water, saline, and isotonic buffer solutions. The swollen solution is heated to 60°C to 100°C and allowed to immerse for a defined period of time to reach a swollen state. It is also desirable to remove unpolymerized monomers contained in the polymer during the swelling process.

The invention is described in more detail below by way of examples, but the invention is not limited to these examples.

### Examples

### [Production example 1 of amorphous carbon-containing ophthalmic hydrogel (contact lens form)]

To a hydrophilic polymer compound solution [1] prepared in the content ratio shown in Table 1, amorphous carbon "MA7" (average particle diameter: 24 nm, manufactured by Mitsubishi Chemical Corporation) was added, and then 20% amorphous carbon dispersion solution [1] was prepared by thoroughly stirring and kneading using a vortex mixer. Next, after performing corona discharge on the surface of the male mold for forming ophthalmic hydrogel, the amorphous carbon dispersion solution [1] was printed using a pad printing machine and allowed to dry naturally at room temperature for 1 hour to form a layer [1] consisting of the amorphous carbon dispersion solution on the male mold surface.

Next, a raw monomer solution, which is a component of Etafilcon (copolymer of 2-hydroxyethyl methacrylate, methacrylic acid, and trimethylolpropane triacrylate) as the hydrogel base material [1], is poured into the female mold for forming ophthalmic hydrogel. A male mold with the layer [1] formed on the surface was fitted, and the temperature was increased in the range of 50°C to 100°C for 25 hours to complete the copolymerization reaction. After cooling to room temperature, the copolymer of contact lens form was removed from the mold and immersed in distilled water at about 80°C for about 4 hours to hydrate and swell, thereby producing the amorphous carbon-containing ophthalmic hydrogel [1] shown in Figs. 1 and 2. The above method was repeated to produce a total of 20 sheets of ophthalmic hydrogel [1].

### [Production examples 2 to 10 of amorphous carbon-containing ophthalmic hydrogel]

In the production example 1 of amorphous carbon-containing ophthalmic hydrogel, 20 sheets ophthalmic hydrogels [2] to [10] were each produced in the same way, except that the hydrophilic polymer compound solution [1] and/or hydrogel base material [1] were replaced with those listed in Table 1 or 2.

### [Production examples A to B of ophthalmic hydrogel]

In the production example 1 of ophthalmic hydrogels-containing amorphous carbon, 20 sheets comparative ophthalmic hydrogels [A] to [B] were each produced in the same way, except that the layer [1] with the amorphous carbon dispersion solution [1] was not formed.

### [Production example 11 of amorphous carbon-containing ophthalmic hydrogel (annular form)]

To a raw monomer solution [1], which is a component of Etafilcon (copolymer of 2-hydroxyethyl methacrylate, methacrylic acid, and trimethylolpropane triacrylate), amorphous carbon "MA7" (average particle diameter: 24 nm, Mitsubishi Chemical Corporation) was further added. The mixture was sufficiently stirred and kneaded using a vortex mixer to prepare a 20% amorphous carbon/raw material monomer solution [1]. Next, the amorphous carbon/raw material monomer solution [1] was injected into the female mold of the annular-formed ophthalmic hydrogel forming mold, the male mold was fitted, and the temperature was increased in the range of 50°C to 100°C for 25 hours to complete the copolymerization reaction. After cooling to room temperature, the copolymer of annular form was removed from the mold and immersed in distilled water at about 80°C for about 4 hours to hydrate and swell, thereby producing the amorphous carbon-containing ophthalmic hydrogel [11] shown in Figs. 3 and 4. The above method was repeated to produce a total of 20 sheets of ophthalmic hydrogel [11].

### [Production example C of ophthalmic hydrogel]

In the production example 11 of amorphous carbon-containing ophthalmic hydrogel, 20 sheets comparative ophthalmic hydrogels [C] were each produced in the same way, except that amorphous carbon was not added.

### [Production example 12 of amorphous carbon-containing ophthalmic hydrogel (oval form)]

To a raw monomer solution [2], which is a component of Polymacon (copolymer of 2-hydroxyethyl methacrylate and ethylene glycol dimethacrylate), 25% by mass of hydrophilic polymer compound solution [1] consisting of 20% by mass of p-HEMA and 80% by mass of 2-hydroxyethyl methacrylate was added. After adding 25% by mass of hydrophilic polymer compound solution [1], amorphous carbon "MA7" (average particle size: 24 nm, manufactured by Mitsubishi Chemical Corporation) was further added to the raw monomer solution prepared by stirring using a vortex mixer. The mixture was sufficiently stirred and kneaded using a vortex mixer to prepare 20% amorphous carbon/raw material monomer solution [2]. Next, the amorphous carbon/raw material monomer solution [2] was injected into the female mold of the oval-formed ophthalmic hydrogel forming mold, the male mold was fitted, and the temperature was increased in the range of 50°C to 100°C for 25 hours to complete the copolymerization reaction. After cooling to room temperature, the copolymer of oval form was removed from the mold and immersed in distilled water at about 80°C for about 4 hours to hydrate and swell, thereby producing the amorphous carbon-containing ophthalmic hydrogel [12] shown in Fig. 5. The above method was repeated to produce a total of 20 sheets of ophthalmic hydrogel [12].

### [Production example D of ophthalmic hydrogel]

In the production example 12 of amorphous carbon-containing ophthalmic hydrogel, 20 sheets of comparative ophthalmic hydrogels [D] were each produced in the same way, except that the hydrophilic polymer compound solution [1] was not added.

### [Adsorption of 5FU]

10 ppm solution of 5FU was prepared using Dulbecco's phosphate buffer as solvent and used as 5FU immersion solution. Each sheet of the ophthalmic hydrogels [1] to [12] and [A] to [D] was immersed in 4 mL of 5FU immersion solution and shaken for 24 hours at room temperature. The ophthalmic hydrogels taken out the 5FU immersion solution were immersed in 4 mL of Dulbecco's phosphate buffer for 24 hours at room temperature to wash off 5FU from the surface of the hydrogel that was not adsorbed on amorphous carbon. The same operation was repeated to produce 20 sheets of 5FU-adsorbed ophthalmic hydrogels for each of the ophthalmic hydrogels [1] to [12] and [A] to [D].

### [Measuring method of 5FU adsorption amount]

A fluorine content in one lot of 10 sheets ophthalmic hydrogels [1] to [12] and [A] to [D], each of which adsorbed 5FU as described above, was determined using an automated combustion halogen and sulfur analysis system (SQ-10 model/HSU-35model manufactured by Yanaco and ICS-2100 model manufactured by Dionex). The content of 5FU was calculated by converting the obtained quantitative fluorine values using the following formula, respectively, and the 5FU adsorption amount was determined. The 5FU adsorption amount by two lots of ophthalmic hydrogels [1] to [12] and [A] to [D], respectively, was calculated and the average values are shown in Table 1. The increase rate in the ophthalmic hydrogels [1] to [5] with respect to the 5FU adsorption amount of ophthalmic hydrogel [A] is shown in Table 1, the increase rate in the ophthalmic hydrogels [6] to [10] with respect to the 5FU adsorption amount of ophthalmic hydrogel [B] is shown in Table 2, the increase rate in the ophthalmic hydrogel [11] with respect to 5FU adsorption amount of ophthalmic hydrogel [C] is shown in Table 3, and the increase rate in the ophthalmic hydrogel [12] with respect to 5FU adsorption amount of ophthalmic hydrogel [D] is shown in Table 4. 5FU adsorption amount (5FU content) (µg/g) = Fluorine content ((µg/g) + Fluorine atomic weight (18.998) × 5FU atomic weight (130.078))

**[Table 1]**

| ophthalmic hydrogel No. | hydrophilic polymer compound solution | | | hydrogel base material | | Fluorine quantitative value (*µ*g/g) | 5FU adsorption amount (*µ*g/g) | increase rate (%) |
|---|---|---|---|---|---|---|---|---|
| | No. | component | | No. | component | | | |
| (1) | (1) | pHEMA | 20wt% | (1) | Etafilcon | 280 | 1917.0 | 19 |
| | | HEMA | 80wt% | | | | | |
| (2) | (2) | pHEMA | 40wt% | (1) | Etafilcon | 285 | 1951.2 | 21 |
| | | HEMA | 60wt% | | | | | |
| (3) | (3) | pHEMA | 60wt% | (1) | Etafilcon | 285 | 1951.2 | 21 |
| | | HEMA | 40wt% | | | | | |
| (4) | (4) | pHEMA | 80wt% | (1) | Etafilcon | 280 | 1917.0 | 19 |
| | | HEMA | 20wt% | | | | | |
| (5) | (5) | pHEMA | 100wt% | (1) | Etafilcon | 285 | 1951.2 | 21 |
| (A) | - | - | | (1) | Etafilcon | 235 | 1608.9 | - |

| | | | | | | | | |
|---|---|---|---|---|---|---|---|---|
| 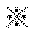 ┌Etafilcon┘ : Copolymer of 2-hydroxyethyl methacrylate, methacrylic acid, and trimethylolpropane triacrylate | | | | | | | | |

**[Table 2]**

| ophthalmic hydrogel No. | hydrophilic polymer compound solution | | | hydrogel base material | | Fluorine quantitative value (*µ*g/g) | 5FU adsorption amount (*µ*g/g) | increase rate (%) |
|---|---|---|---|---|---|---|---|---|
| | No. | component | | No. | component | | | |
| (6) | (1) | pHEMA | 20wt% | (2) | Polymacon | 200 | 1369.3 | 11 |
| | | HEMA | 80wt% | | | | | |
| (7) | (2) | pHEMA | 40wt% | (2) | Polymacon | 190 | 1300.8 | 6 |
| | | HEMA | 60wt% | | | | | |
| (8) | (3) | pHEMA | 60wt% | (2) | Polymacon | 205 | 1403.5 | 14 |
| | | HEMA | 40wt% | | | | | |
| (9) | (4) | pHEMA | 80wt% | (2) | Polymacon | 200 | 1369.3 | 11 |
| | | HEMA | 20wt% | | | | | |
| (10) | (5) | pHEMA | 100wt% | (2) | Polymacon | 210 | 1437.7 | 17 |
| (B) | - | - | | (2) | Polymacon | 180 | 1232.3 | - |

| | | | | | | | | |
|---|---|---|---|---|---|---|---|---|
| 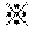 ┌Po3ymacon┘ : Copolymer of 2-hydroxyethyl methacrylate and ethylene glycol dimethacrylate | | | | | | | | |

**[Table 3]**

| ophthalmic hydrogel No. | raw material monomer solution | | Fluorine quantitative value (*µ*g/g) | 5FU adsorption amount (*µ*g/g) | increase rate (%) |
|---|---|---|---|---|---|
| | No. | component | | | |
| (11) | (1) | Etafilcon | 255 | 1745.8 | 8.5 |
| (C) | (1) | Etafilcon | 235 | 1608.9 | - |

| | | | | | |
|---|---|---|---|---|---|
| 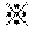 ┌Etaflicon┘ : Copolymer of 2-hydroxyethyl methacrylate, methacrylic acid, and trimethylolpropane triacrylate | | | | | |

**[Table 4]**

| ophthalmic hydrogel No. | hydrophilic polymer compound solution | | | raw material monomer solution | | Fluorine quantitative value (*µ*g/g) | 5FU adsorption amount (*µ*g/g) | increase rate (%) |
|---|---|---|---|---|---|---|---|---|
| | No. | component | | No. | component | | | |
| (12) | (1) | pHEMA | 20wt% | (2) | Polymacon | 205 | 1403.5 | 13.9 |
| | | HEMA | 80wt% | | | | | |
| (D) | - | - | | (2) | Polymacon | 180 | 1232.3 | - |

| | | | | | | | | |
|---|---|---|---|---|---|---|---|---|
| 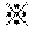 ┌Polymacon┘ : Copolymer of 2-hydroxyethyl methacrylate and ethylene glycol dimethacrylate | | | | | | | | |

The results in Tables 1 to 4 show that according to the ophthalmic hydrogels containing amorphous carbon [1] to [12], the increase rates of 5FU adsorption amount were improved compared to the ophthalmic hydrogels [A] to [D] that did not contain amorphous carbon, respectively. The results indicate that the hydrogels containing amorphous carbon according to one embodiment of the present invention can improve the adsorption rate of 5FU compared to hydrogels that do not contain amorphous carbon.

### Industrial Applicability

In the hydrogel of the present invention, when the hydrogel is for ophthalmic hydrogel, wearing the ophthalmic hydrogel, for example, 5FU that remains in the tear fluid is adsorbed to the amorphous carbon, so that 5FU is removed from the tear fluid. It is expected to prevent the occurrence of lacrimation, which is one of the side effects.

### Cross-reference of related applications

This application claims the benefit of priority to Japanese Patent Application No. 2021-179262, filed November 2, 2021, the disclosure of which is incorporated herein by reference in its entirety.

## Claims

1. A hydrogel comprising amorphous carbon.

2. The hydrogel according to claim 1, wherein the hydrogel is an ophthalmic hydrogel.

3. The hydrogel according to claim 2, wherein the ophthalmic hydrogel is in a contact lens form, and the amorphous carbon is arranged in a circular pattern on the surface and/or inside except for the optical area of the hydrogel.

4. The hydrogel according to claim 2, wherein the ophthalmic hydrogel is in an annular or approximately oval form, and the amorphous carbon is uniformly arranged throughout the surface and/or inside of the hydrogel.

5. The hydrogel according to any one of claims 1 to 4, wherein the amorphous carbon is physically capable of adsorbing a predefined compound.
